# EUROPEAN PATENT SPECIFICATION

(11) **EP 2 682 748 B1**
(45) Date of publication and mention of the grant of the patent: **18.03.2015**
(21) Application number: 12718746.6
(22) Date of filing: 05.03.2012
(51) Int. Cl.: G01N 33/50

(54) **METHOD OF LABELING NEURONS AND ITS APPLICATION**
VERFAHREN ZUR MARKIERUNG VON NEURONEN UND DESSEN ANWENDUNG
METHODE DE MARQUAGE DES NEURONES ET SON UTILISATION

(30) Priority: 03.03.2011 PT 11105555
(43) Date of publication of application: 08.01.2014
(73) Proprietor: Universidade do Minho, 4704-553 Braga (PT)
(72) Inventor: MEIRELES PINTO, Luísa Alexandra, P-4710-057 Braga (PT); CARVALHO SOUSA, Nuno Jorge, P-4710-057 Braga (PT); RESTOLHO MATEUS PINHEIRO, António Maria, P-4710-057 Braga (PT); SEIÇA BESSA PEIXOTO, João Miguel, P-4710-057 Braga (PT); DIAS MORAIS, Mónica Susana, P-4710-057 Braga (PT)
(74) Representative: Ferreira, Maria Silvina
(86) International application number: PCT/IB2012/051032
(87) International publication number: WO 2012/117387

(56) References cited:
- ROSALIA CRUPI ET AL: "Melatonin treatment mimics the antidepressant action in chronic corticosterone-treated mice", JOURNAL OF PINEAL RESEARCH, 1 May 2010 (2010-05-01), pages NO-NO, XP55028815, ISSN: 0742-3098, DOI: 10.1111/j.1600-079X.2010.00775.x

## Description

### Field of Invention

The present invention integrates in the Health Sciences Technology domain, specifically in the Neurosciences domain.

### Background of the invention

The unique phenomena of adult neurogenesis encompasses the generation and incorporation of newborn neurons in pre-existing networks of the adult brain and constitutes the most interesting case of brain neuroplasticity. Adult neurogenesis occurs indisputably in two brain regions that give rise to two different types of neurons: the subgranular zone (SGZ) of the dentate gyrus (DG), where glutamatergic neurons are generated; and the subependymal zone (SEZ), where different classes of GABAergic neurons of the olfactory bulbs (OB) are generated. Recently, scientific research has started to study all the components of the neurogenic process, trying to unveil its functional importance through different techniques that allow to measure neurogenesis. Notably, the process of neurogenesis is dysfunctional in several diseases.

Several strategies have been developed to study adult neurogenesis. In order to successfully study the activity of stem-cells *in vivo,* it is necessary to identify proliferative cells and stably label their cell progeny. In rodents, transgenic animal models, retroviral cell proliferation labeling and immunohistochemistry for bromodeoxyuridine (BrdU) are robust technical approaches to study the generation and migration of newly-born cells in the adult brain. However, most of these techniques are not applicable in humans due to its high invasiveness.

Cell labeling with BrdU is a widely used and prevalent method for studying proliferation and neurogenesis *in vivo.* When injected systemically, BrdU (a thymidine analog) is incorporated in the DNA of proliferative cells, which are synthesizing new DNA, allowing the detection of these cells along their post-mitotic life cycle. This technique is pivotal for the identification of the source of neuronal cells formed *de novo* in the SGZ and SEZ adult brain regions. Additional studies with multiple cell stainings, where BrdU immunohistochemistry and confocal microscopy were used, revealed that newborn neurons formed in the adult brain express a series of cell markers that reflect their maturation status. The identification of the maturation state of these newly-born neurons can thus be done by multiple labeling using BrdU and neuronal markers, such as NeuN. Other alternative methods were developed to measure proliferation *in vivo* using cell division markers, such as PCNA and Ki67, which are good markers for cells undergoing cell cycle. However, these cell markers are not suitable to detect the cell progeny (in contrast to BrdU, that is maintained in the progeny of the proliferating cells), as when the cells express mature neuronal markers, they already left the cell cycle.

Hence, other methods are necessary to detect adult neurogenesis and to unequivocally confirm its existence with BrdU labeling. Moreover, BrdU labeling does not allow for morphological and connectivity studies in newborn neurons of the adult brain, such as studies of the dendritic arborization and spine morphology. Therefore, an alternative cell labeling technique with retrovirus has been developed, which has become the most used method to study adult neurogenesis. Viral vectors are able to integrate in the genome of proliferative cells resulting in the permanent labeling of its entire progeny. In virtue of such characteristic, this method proved to be useful also to study the morphological and physiological development of newly-born neurons, along all their maturation phases, a feature not feasible with the BrdU immunohistochemistry technique. In fact, the detection of newborn neurons by retroviral vectors provides several other advantages, in comparison to BrdU immunohistochemistry. For example, retroviral labeling allows distinguishing between cell division and DNA repair, since viral integration in the host genome can only occur once the cell membrane has been disrupted during division. Furthermore, the method referent to the invention here described, allows for the labeling of the entire progeny of a proliferative cell, independently of the number of cell divisions, contrarily to BrdU staining that is diluted along divisions. Since the blood brain barrier is an obstacle for the entry of retroviral vectors in the brain, these viruses must be injected directly in the region of interest by stereotaxic surgeries, causing brain lesions associated to the canula implantation along with possible inflammatory reactions. This traumatic and pro-inflammatory method can induce significant alterations in neurogenesis, which raises some constrains and putative confounding factors when studying the neurogenic process. Also, in the central nervous systems it was already reported that the incorporated retroviral construct might be silenced, and thus not be expressed in the daughter cells, bringing additional cautions to this method. Due to the possibility of occurring gene silencing in the viral construct of stem cells, it is not possible to assure that all the cell progeny is labeled. In addition, it is not known if retroviruses, once integrated, are subjected to epigenetic regulation that can undermine a clear interpretation of the results. Nevertheless, retroviral labeling is still considered the most robust method to study adult neurogenesis in the brain. However, considering the many disadvantages above mentioned, it is crucial to be cautious when interpreting neurogenesis measurements in the healthy brain as well as in the "diseased" brain. Therefore, there is a need of finding new and more consistent methods to study with greater accuracy this phenomena occurring in the adult brain.

Importantly, the neurogenic process, including proliferation of the stem cells and survival of the newly-born neurons, may be affected by different pathological conditions. In fact, previous studies showed that imbalances in adult hippocampal neurogenesis might underlie the pathophysiology of depression, as well as the action of antidepressant drugs, giving rise to the "neurogenic hypothesis of depression". Alterations in neuronal structure both in the hippocampus and in the prefrontal cortex are known to be key processes in the pathophysiology of depression. Interestingly, there is a strong contribution of the synaptic plasticity and neuronal connectivity to the development and recovery of a depressive behavior. In fact, synaptic connections, disrupted in depressed individuals are reestablished by antidepressants treatment, such as fluoxetine and imipramine, promoting the recovery of the neuronal circuits.

In the majority of these studies, the assessment of the dendritic arborization and spine morphology have been performed by three-dimensional morphometric analysis, using computer-automated virtual reconstructions, in the hippocampus and cortex. Despite being possible to study neuronal morphology in areas of interest, this technique does not allow to distinguish older pre-existing neurons from newly generated neurons, which are specifically affected by the disease and treatment. Therefore, it becomes fundamental to design an alternative method that allows studying the morphology and connectivity specifically of newborn neurons, by being able to identify them. A good alternative would be to use retroviral labeling of newborn cells and simultaneously to study neuronal structure, including dendritic and axonal projections. However, due to the several disadvantages mentioned before, using retroviruses requires many precautions in the subsequent interpretation of the obtained results, especially in pathological scenarios. Besides that, this method involves a surgery and thus it is invasive, making impossible to use this method in humans. For this reason, it is valuable to have a technique that allows simultaneously labeling the newborn neurons and studying their morphology, avoiding problems such as lesions and inflammatory reactions in the brain region of interest. In this sense, the innovative and non-invasive method of the present invention allows obtaining these objectives with success through a method that combines immunohistochemistry for BrdU with Golgi-Cox impregnation in neurons.

### Summary of the invention

The methodology for labeling of neurons described in the present invention combines immunohistochemistry for bromodeoxyuridine (BrdU) with the Golgi-Cox impregnation technique. With this simper and non-invasive method it is possible to:
- Determine the three-dimensional structure of the dendritic arborization and the spines shape of newborn neurons that are generated in a specific time point, in the hippocampus of adult animals, in physiological and pathological conditions, such as in depression;
- Study with extreme precision and in a non-invasive manner the neurogenic process in the adult brain.

The present invention describes a methodology for labeling of neurons comprising simultaneously:
- Golgi-Cox impregnation technique;
- Technique of immunohistochemistry for bromodeoxyuridine (BrdU) on the same sample.

In a preferred embodiment of the present invention the methodology for labeling of neurons is characterized by comprising the following steps:
a) Establish the Golgi-Cox impregnation technique in neurons samples;
b) Establish an immunohistochemistry for BrdU in the same samples previously impregnated with Golgi-Cox;
c) Analyses of the samples.

In a most preferred embodiment of the present invention the step a) mentioned above comprises the following steps:
i. placing the sample in Golgi-Cox solution and maintain it in the dark for at least 15 days;
ii. placing the sample in a 30% sucrose solution, previously refreshed;
iii. maintaining the sample under refrigeration at a temperature of 4 ° C in the dark for 2-5 days;
iv. dipping the sample in distilled water for 15 minutes, then in ammonium hydroxide for 5 minutes in the dark;
v. washing the samples with distilled water and dipping the samples in the Kodak fixing solution;
vi. washing the samples with distilled water and dipping the samples in phosphate buffered saline solution.

In one further preferred embodiment of the present invention in step ii) of step a) mentioned above the solution is cooled to a temperature of 4°C.

In one embodiment further preferably of the present invention in step v) of step a) the samples are submerged in the fixing solution for at least 20 minutes.

In another preferred embodiment of the present invention the step b) mentioned above comprises the following steps:
i. placing the samples in citrate buffer solution;
ii. heating the samples for 5 minutes;
iii. cooling the specimens to room temperature;
iv. dipping the samples in Tris buffer solution three times;
v. incubate the samples with primary antibody for BrdU;
vi. removing the primary antibody solution and dipping the samples three times in TBS, 10 minutes each;
vii. incubating the sample with secondary antibody for 2 hours at room temperature;
viii. removing the secondary antibody and dipping the samples in TBS three times;
ix. incubating the samples in DAPI at the concentration of 1µg/ml in a solution of TBS for 10 minutes at room temperature and then the samples were dipped 3 times in TBS, 3 minutes each.

In one further preferred embodiment of the present invention in step i) of step b) mentioned above the buffer solution has a concentration of 10 mM and pH 6.

In one embodiment further preferably of the present invention the step ii) of step b) occurs at a power between 750 and 900 watts.

In one further preferred embodiment of the present invention in step iv) of step b) the samples remain immersed in Tris buffer for 10 minutes each time.

In one embodiment further preferably of the present invention in step v) of step b) the samples are incubated overnight at 4°C.

In one further preferred embodiment of the present invention in step b) the primary antibody for BrdU is used at a dilution of 1:50.

In one embodiment further preferably of the present invention in step viii) of step b) the samples remain immersed in TBS 10 minutes each wash.

In another preferred embodiment of the present invention the secondary antibody is used at the dilution of 1:1000.

The present invention also describes the application of the methodology described in the analysis of the three-dimensional structure of the dendritic arborization of the neurons and their spine shape.

In a preferred embodiment of the use of this methodology this invention is applied in physiological or pathological conditions in which neurogenesis and synaptic plasticity may be affected.

### General Description

Observation of neuronal dendritic structure in combination with determination of specific neuronal phenotype is actually a great challenge. In the present invention we developed a novel method that combines bromodeoxyuridine (BrdU) immunohistochemistry with a Golgi-impregnation technique in neurons. With this simple and non-invasive method, we are able to determine the three-dimensional structure of the dendritic arborization and spine shape of specifically newly-born neurons in the hippocampal formation of adult animals, in physiological and pathological conditions, such as in depression.

To develop and implement this method, several experimental steps were needed comprising:
- establishing the Golgi-Cox impregnation method of neurons in brain slices of rats in physiological and pathological conditions;
- establishing the BrdU immunohistochemistry in the same brain slices where we previously performed the Golgi impregnation;
- performing confocal microscope and stereological analyses to detect the combined labeling of BrdU and Golgi-Cox impregnation.

The detailed steps of the implementation of the methodology of the present invention are:

### 1-Golgi-Cox impregnation in neurons

Rats are deeply anaesthetized with sodium pentobarbital (Eutasil, 60mg/Kg i.p. per rat with the weight of 300-500grams; Ceva Saúde Animal, Portugal) and perfused with 0.9% saline immediately after 4 weeks of the BrdU injections (5 injections i.p in 5 consecutive days, 100mg/kg). All brains are removed, dropped into Golgi-Cox solution and kept in the dark for 15 days. Next, brains are transferred to 30% sucrose solution (previously cooled in the refrigerator at 4°C) and kept in the refrigerator for 2 to 5 days in the dark until they sink. Brains are sectioned using a vibratome (Microm HM 650V) after drying on wipe paper and fixing them onto the vibratome platform using cyanoacrylate (sections with the thickness of 200µm). Sections are transferred to 24-well multiwell plate (Nunc) filled with distilled water for 15 minutes and then are dipped in ammonium hydroxide (Sigma Aldrich) for 5 minutes in the dark. Next, sections are washed with distilled water twice, 10 minutes each, and dipped in Kodak Fix solution (Rapid fixer; Sigma Aldrich) for 20 minutes. We perform 2 more washes in distilled water, 10 minutes each, and next dip sections in PBS 1X, keeping them cool in the refrigerator at 4°C.

### 2-Immunohistochemistry for BrdU

Immediately after finishing the procedure for Golgi-Cox staining, sections are transferred to 6-well multiwell plates (maximum 10 sections per well) filled with citrate buffer (10mM; pH=6). We then place the lid loosely on the plates and heat the sections for 5 minutes in the microwave at medium temperature (potency between 750 and 900). Multiwell plates are removed from the microwave and placed at room temperature to allow the sections to cool for around 15 minutes. Sections are rinsed in TBS for 3 times, each 10 minutes, and next are incubated with primary BrdU antibody (rat anti-mouse from Novocastra) at the dilution of 1:50 in 0.5% Triton®-X 100 and 10% NGS solution overnight at 4°C (500µl of solution per well). In the next day, the primary antibody solution is removed and sections are rinsed with TBS for 3 times, 10 minutes each. Sections are then incubated with secondary antibody (anti-mouse Alexa Fluor 488 or Alexa Fluor 546 from Invitrogen) at the dilution of 1:1000 in 0.5% Triton®-X 100 and 10% NGS solution for 2 hours at room temperature. The secondary antibody is removed and sections are rinsed in TBS for 3 times, 10 minutes each. Finally, sections are incubated in DAPI at the concentration of 1µg/ml in TBS solution for 10 minutes at room temperature and then are rinsed again in TBS for 3 times, 3 minutes each. Sections are mounted in superfrost slides (SuperFrost Ultra Plus from Menzel-Gläser; 3 sections per slide) using Vectashield mounting medium (Vector Labs). Slides are allowed to lie flat and air dry for at least 48 hours in a dark cool place (4°C).

### 3- Confocal microscopy analyses

After allowing the slides to completely dry, imaging of neurons double stained for Golgi-Cox and BrdU localized in the DG of the hippocampus are carried out using an Olympus FV1000 laser scanning confocal microscope (emission wavelength 488 or 594 and brightfield) at high magnification (40x). Sections are optically sectioned using 1-2µm intervals and cells are rotated in orthogonal planes to verify double labeling.

### 4- Stereological analyses

Dendritic arborization and spines are analyzed in the DG of the hippocampus. For each selected neuron that shows co-localization of Golgi-Cox with BrdU, all branches of the dendritic tree are reconstructed at x600 (oil) magnification using a motorized microscope (Axioplan 2; Carl Zeiss) and Neurolucida software (Microbrightfield) with the AutoNeuron extension module. Three-dimensional analyses of the reconstructed neurons are performed using NeuroExplorer software (Microbrightfield).

### Brief description of the figures

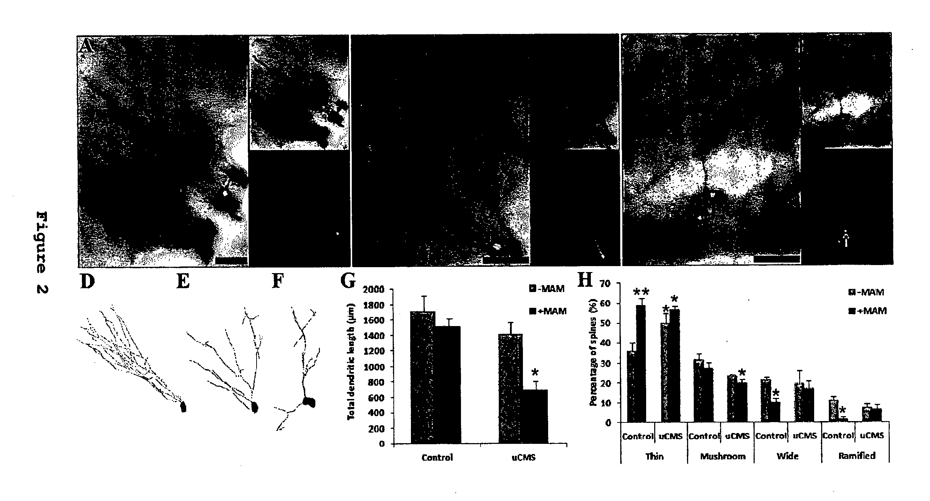

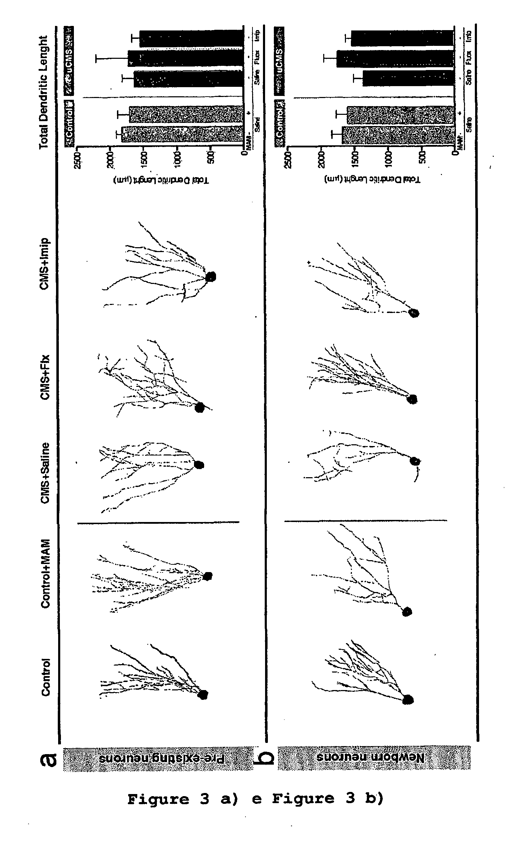

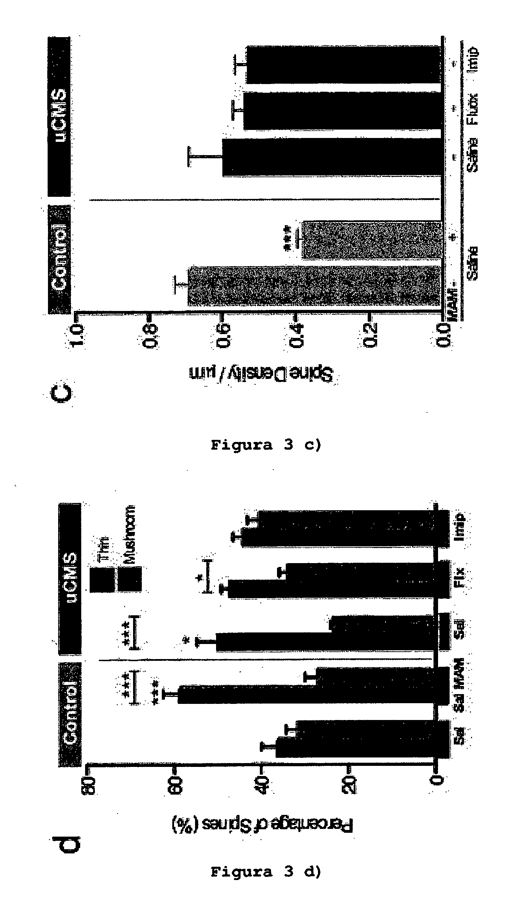

For easy understanding of the present invention we attached figures that represent preferred realizations of the invention which, however, are not intended to limit the object of the present invention.

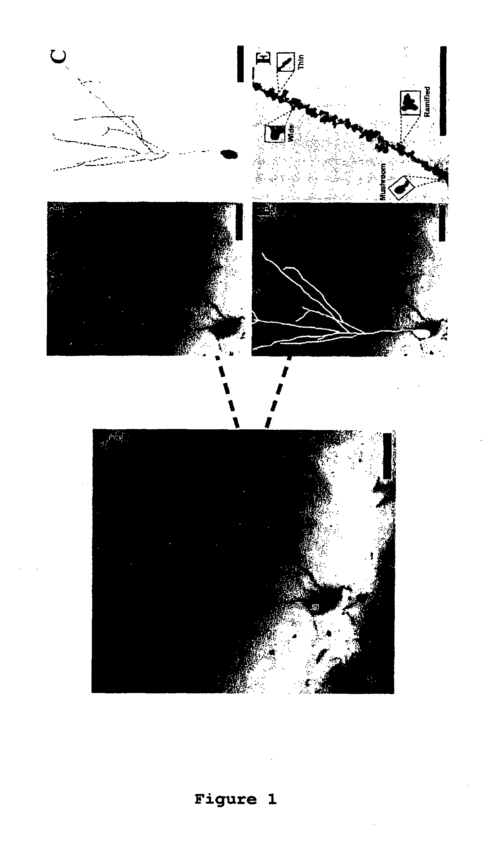

**Figure 1****. Three-dimensional analysis of neurons double-labeled with BrdU and Golgi-Cox**
   Three-dimensional morphometric analysis of a Golgi-impregnated neuron co-labeled with BrdU (depicted with green dot) and Dapi (staining of nuclei depicted with blue dots) **(A)** using computer-assisted reconstructions. Neuronal reconstruction was performed using a motorized microscope and Neurolucida software with the automatic AutoNeuron extension module directly from the confocal image (red color in **B** and **C**) and using manual reconstruction (white color in **D**). Different colors on **C** depict distinct dendritic branches and black arrows in **B** and **D** depict the differences detected between the automatic and manual reconstructions. Image **E** depicts a neuronal segment showing different spine types. Scale bars: 50µm
**Figure 2****. Confocal images and three-dimensional morphometric analysis of neurons double-labeled with BrdU and Golgi-Cox**
   **(A, B, C)** Confocal images of three dentate gyrus neurons double-labeled with BrdU (depicted with green dots) and Golgi-Cox (black staining). BrdU was administered for five consecutive days to 4 months old Wistar rats non-stressed and injected with saline for two weeks (Control) (**A**) or exposed to unpredictable chronic mild stress (uCMS) and injected with saline (**B**) or methyazoxymethanol (uCMS+MAM) (**C**) in the last two weeks of the stress protocol. Immunohistochemical analyses were performed 4 weeks after the injections. Nuclei (depicted with blue dots) were stained with Dapi. (**D, E, F**) Three-dimensional morphometric reconstruction analysis of the Golgi-impregnated dentate granule neurons double-labeled with BrdU shown in A (**D**) in B (**E**) and in C (**F**). (**G**) Graph showing the total dendritic length of newborn dentate granule neurons in the subgranular zone of different experimental groups (control and uCMS exposed rats untreated (-MAM) and treated with MAM (+MAM)). (**H**) Graph showing the percentage of different types of spines (thin, mushroom, thick and ramified) present in newborn dentate granule neurons in the subgranular zone of different experimental groups (control and uCMS exposed rats untreated (-MAM) and treated with MAM (+MAM)). Data represented as mean±se.m. Scale bars: 50 m
**Figura** 3. **Exposure to chronic mild stress causes long-term neuromorphological alterations in newborn neurons of the hippocampus of adult rats.**
   (**a**) Structural changes induced by chronic mild stress (CMS) exposure are reverted at long-term and do not associate to the observed long-lasting behavioral deficits. (**b** and **c**) CMS exposure does not have long-term impact on the dendritic arborisation (**b**), neither in the global spine density of newborn neurons in the hippocampal dentate gyrus (**c**). (**d**) Newborn granule neurons of rats previously exposed to CMS present increase percentage of thin spines and these alterations are attenuated in animal groups treated with the antidepressants fluoxetine and imipramine.
   Noteworthy that the pre-existing neurons in **a** were analyzed only for the Golgi impregnation labeling and the newly-born neurons in **b** were labeled with Golgi and BrdU simultaneously (Immuno-golgi method here described). This novel technique allowed analyzing the neurons generated de novo in the adult hippocampus, showing morphological alterations in the spines that happen specifically in these newly-born neurons and not in the pre-existing neurons. Thus, this novel technology allowed for the first time to distinguish between pre-existing and newly-born neurons and detect alterations specifically in this last type of neurons.
   * Denotes the effect of CMS and MAM; # Denotes antidepressant effect. *P<0.05, **P<0.01, ***P<0.001; n=10 per group.

### Detailed description of the invention

To study stem cell activity *in vivo,* it is necessary to identify proliferating cells, but also to stably label their progeny so that other components of neurogenesis can be studied. Herein we describe a novel method developed by us that successfully allows the accomplishment of these objectives by combining bromodeoxyuridine (BrdU) immunohistochemistry labeling with Golgi-impregnated neuron analysis (**Fig. 1**). With this new method we can trace newborn neurons using BrdU labeling and study their structure, including dendritic arborization and spine shape, by simultaneous analysis of Golgi-impregnated neurons. To validate this method, we studied neurons that showed co-labeling of BrdU and Golgi-Cox staining using confocal microscopy (**Figs. 1** and **2**) and performed elaborated three-dimensional morphometric analysis of the Golgi-impregnated neurons using computer-assisted reconstructions (**Figs. 1** and **2**). All branches of the dendritic tree of the selected neurons were reconstructed using a motorized microscope and Neurolucida software with the AutoNeuron extension module (**Fig. 1B, C**) and using manual reconstruction without the AutoNeuron extension (**Fig. 1D**). As we can show in the figures, no significant differences in total dendritic length per neuron were found between the automatic AutoNeuron and manual reconstructions, thus showing that distinct reconstruction strategies can be used to study the structure of these neurons. A three-dimensional analysis of the reconstructed neurons was performed using NeuroExplorer software (Microbrightfield). This method also allows the analysis of spine types (**Fig. 1E**) which provides information on neuronal connectivity and synaptic plasticity.

To validate this method, we studied the structure of newborn neurons in the dentate gyrus (DG) of control rats (**Figs. 1** and **2**) and compared to the DG neurons of rats displaying depressive-like symptoms after exposure to chronic mild stress (CMS) and/or to the administration of methyazoxymethanol (MAM), an alkylating agent that arrests cellular proliferation. It is well established that proliferation of neuronal progenitors and survival of newborn neurons can be regulated by physiological and pathological conditions. Indeed, previous studies have proposed that imbalances in adult neurogenesis in the hippocampus could be involved in the pathophysiology of depression and in the actions of antidepressant drugs. Additionally, it recently emerged that the expression of depressive-like behavior in CMS rats is associated with decreased proliferation in the hippocampus, but also with dendritic atrophy and synaptic loss in this brain region. Strikingly, there is a potential contribution of synaptic plasticity and neuronal connectivity to the development of, and recovery from, depressive-like behavior, as these synaptic connections can be restored by antidepressants, facilitating the rewiring of neural circuits. In most of these studies, the assessment of dendritic arborization and spine shape of neurons was performed by three-dimensional morphometric analysis of Golgi-impregnated neurons in the hippocampus without making possible to distinguish between old non-affected neurons and newly-born neurons that were affected by the disease or by the treatment. The distinction between these two types of neurons is of extreme importance to convincingly study the effect of a disease and its treatment in the newly formed neurons of the brain. These studies were not possible to perform until now as the researchers didn't have any available method that could allow analyzing specifically the newly-born neurons affected by the disease and treatment. With the new method of the present invention we can label the neurons that were born during the period of CMS using staining for BrdU and study simultaneously their cell shape, including dendritic processes by Golgi-Cox staining.

Thus, we could compare specifically the morphology of newborn neurons in rats subjected to unpredictable CMS with and without MAM administration (**Fig. 2B** and **C**) with newborn neurons of control rats (**Fig. 2A**). We show here that newborn neurons from the DG of rats subjected to CMS without blockage of neurogenesis are able to recover morphologically (**Fig. 2B, E, G** and **H**) and are comparable to newborn neurons of control untreated rats (**Fig. 2A, D, G** and **H**). These findings also show for the first time that the newborn neurons from the DG of rats subjected to CMS and injected with MAM are severely compromised and are not able to recover in terms of cell shape and dendritic arborization even one month after stress exposure (**Fig. 2C, F, G** and **H**).

Given that neuroplasticity, or its failure, is crucial in several neuropathological processes, it becomes extremely relevant to compare the neuronal structure of newly-born cells in comparison to those of previously existent cells. So far, the analysis of the dendritic and synaptic structure of newly-born neurons could only be achieved by retroviral labeling. However, this method has several disadvantages, in comparison to our novel method, as for example being and invasive method that causes lesions and local inflammatory reactions that make it less suitable for use in *in vivo* studies. These possible lesions and inflammation reactions may induce alterations on neurogenesis, thus raising some cautions when using this method to study stem cell activity in the brain in a neurophatological context. Moreover, this approach only permits the study of the region where the virus was injected and does not allow for precise temporal resolution of proliferation nor for the comparison of the neuronal morphology between dividing and non-dividing cells. All these disadvantages are overcome by the method of the present invention.

Noteworthy that standard histopathological techniques used to label neurons do not stain dendrites and spines and thus may miss aberrant dendritic branching and synaptic loss in neurodegenerative processes. In contrast, the Golgi silver impregnation is a simple and valuable method that provides detailed information on neuronal morphology of the neurons allowing the detection of subtle neuronal damages or degeneration. As shown with the present invention, if the Golgi-Cox impregnation is combined with BrdU staining, this technique will further allow distinguishing whether these changes target specific neuronal populations (e.g. in the present study, whether they affect differently old versus newly born neurons). Obviously, the applicability of this method is much broader, as the combination of other markers and Golgi will allow the analysis of the neuronal dendritic structure with its phenotypic characterization in a wide spectrum of experimental conditions. Most importantly is the fact that this method can be applied to humans, as it is non-invasive, in contrast to the most used methods nowadays, more precisely the retroviral injections method.

In summary, this novel simple and non-invasive method will be a useful tool to study the fine neuronal structure in phenotypically characterized neurons in both physiological and pathological conditions. The methodology of the present invention, is useful for all those pathological conditions in which neurogenesis and dendritic/synaptic plasticity might be affected, such as in depression, Alzheimer's disease and schizophrenia.

### References

1. Alvarez-Buylla, A., Garcia-Verdugo, J.M. & Tramontin, A.D. A unified hypothesis on the lineage of neural stem cells. Nature reviews 2, 287-293 (2001).
2. Seri, B., Garcia-Verdugo, J.M., Collado-Morente, L., McEwen, B.S. & Alvarez-Buylla, A. Cell types, lineage, and architecture of the germinal zone in the adult dentate gyrus. J Comp Neurol 478, 359-378 (2004).
3. Seri, B., Garcia-Verdugo, J.M., McEwen, B.S. & Alvarez-Buylla, A. Astrocytes give rise to new neurons in the adult mammalian hippocampus. J Neurosci 21, 7153-7160 (2001).
4. Nilsson, M., Perfilieva, E., Johansson, U., Orwar, O. & Eriksson, P.S. Enriched environment increases neurogenesis in the adult rat dentate gyrus and improves spatial memory. Journal of neurobiology 39, 569-578 (1999).
5. Petreanu, L. & Alvarez-Buylla, A. Maturation and death of adult-born olfactory bulb granule neurons: role of olfaction. J Neurosci 22, 6106-6113 (2002).
6. Lemasson, M., Saghatelyan, A., Olivo-Marin, J.C. & Lledo, P.M. Neonatal and adult neurogenesis provide two distinct populations of newborn neurons to the mouse olfactory bulb. J Neurosci 25, 6816-6825 (2005).
7. Dranovsky, A. & Hen, R. Hippocampal neurogenesis: regulation by stress and antidepressants. Biological psychiatry 59, 1136-1143 (2006).
8. Pittenger, C. & Duman, R.S. Stress, depression, and neuroplasticity: a convergence of mechanisms. Neuropsychopharmacology 33, 88-109 (2008).
9. van Praag, H., et al. Functional neurogenesis in the adult hippocampus. Nature 415, 1030-1034 (2002).
10. Yamaguchi, M., Saito, H., Suzuki, M. & Mori, K. Visualization of neurogenesis in the central nervous system using nestin promoter-GFP transgenic mice. Neuroreport 11, 1991-1996 (2000).
11. Lois, C., Garcia-Verdugo, J.M. & Alvarez-Buylla, A. Chain migration of neuronal precursors. Science (New York, N.Y 271, 978-981 (1996).
12. Seki, T. & Arai, Y. Temporal and spacial relationships between PSA-NCAM-expressing, newly generated granule cells, and radial glia-like cells in the adult dentate gyrus. The Journal of comparative neurology 410, 503-513 (1999).
13. Doetsch, F. & Alvarez-Buylla, A. Network of tangential pathways for neuronal migration in adult mammalian brain. Proceedings of the National Academy of Sciences of the United States of America 93, 14895-14900 (1996).
14. Wichterle, H., Garcia-Verdugo, J.M. & Alvarez-Buylla, A. Direct evidence for homotypic, glia-independent neuronal migration. Neuron 18, 779-791 (1997).
15. Kuhn, H.G., Dickinson-Anson, H. & Gage, F.H. Neurogenesis in the dentate gyrus of the adult rat: age-related decrease of neuronal progenitor proliferation. J Neurosci 16, 2027-2033 (1996).
16. Palmer, T.D., Willhoite, A.R. & Gage, F.H. Vascular niche for adult hippocampal neurogenesis. The Journal of comparative neurology 425, 479-494 (2000).
17. Kempermann, G., Kuhn, H.G. & Gage, F.H. More hippocampal neurons in adult mice living in an enriched environment. Nature 386, 493-495 (1997).
18. Gould, E., McEwen, B.S., Tanapat, P., Galea, L.A. & Fuchs, E. Neurogenesis in the dentate gyrus of the adult tree shrew is regulated by psychosocial stress and NMDA receptor activation. J Neurosci 17, 2492-2498 (1997).
19. Abrous, D.N., Koehl, M. & Le Moal, M. Adult neurogenesis: from precursors to network and physiology. Physiol Rev 85, 523-569 (2005).
20. Ming, G.L. & Song, H. Adult neurogenesis in the mammalian central nervous system. Annu Rev Neurosci 28, 223-250 (2005).
21. van Praag, H., Kempermann, G. & Gage, F.H. Running increases cell proliferation and neurogenesis in the adult mouse dentate gyrus. Nature neuroscience 2, 266-270 (1999).
22. Bessa, J.M., et al. The mood-improving actions of antidepressants do not depend on neurogenesis but are associated with neuronal remodeling. Molecular psychiatry 14, 764-773, 739 (2009).
23. Spiga, S., et al. Simultaneous Golgi-Cox and immunofluorescence using confocal microscopy. Brain Struct Funct 216, 171-182.
24. Fisher, R. & Boylan, M. Tachykinin-immunoreactive neurons in developing feline neostriatum: somatodendritic morphogenesis demonstrated by combined immunohistochemistry/Golgi impregnation-gold toning. Dev Neurosci 33, 75-84.
25. Cerqueira, J.J., Taipa, R., Uylings, H.B., Almeida, O.F. & Sousa, N. Specific configuration of dendritic degeneration in pyramidal neurons of the medial prefrontal cortex induced by differing corticosteroid regimens. Cereb Cortex 17, 1998-2006 (2007).
26. Airan, R.D., et al. High-speed imaging reveals neurophysiological links to behavior in an animal model of depression. Science 317, 819-823 (2007).
27. Malberg, J.E., Eisch, A.J., Nestler, E.J. & Duman, R.S. Chronic antidepressant treatment increases neurogenesis in adult rat hippocampus. J Neurosci 20, 9104-9110 (2000).
28. Price, J., et al. Labelling neural precursor cells with retroviruses. Gene Ther 1 Suppl 1, S4-5 (1994).
29. DeCarolis, N.A. & Eisch, A.J. Hippocampal neurogenesis as a target for the treatment of mental illness: a critical evaluation. Neuropharmacology 58, 884-893.
30. Landgren, H. & Curtis, M.A. Locating and labeling neural stem cells in the brain. J Cell Physiol 226, 1-7.
31. Goncalves, L., et al. Neuropathic pain is associated with depressive behaviour and induces neuroplasticity in the amygdala of the rat. Exp Neurol 213, 48-56 (2008).
32. Redila, V. A. & Christie, B. R. Exercise-induced changes in dendritic structure and complexity in the adult hippocampal dentate gyrus. Neuroscience 137, 1299-307 (2006).
33. Crupi, R., et al., Melatonin treatment mimics the antidepressant action in chronic corticosterone-treated mice. J. Pineal Res. 2010, 49: 123-129.

The following claims define the present invention.

## Claims

1. Methodology for labeling of neurons **characterized by** combining simultaneously the Golgi-Cox impregnation technique and the technique of immunohistochemistry for bromodeoxyuridine (BrdU) on the same sample.

2. Methodology according to the previous claim **characterized by** comprising the following steps:
a) establish the Golgi-Cox impregnation technique in neurons samples;
b) establish an immunohistochemistry for BrdU in the same samples previously impregnated with Golgi-Cox;
c) analyses of the samples.

3. Methodology according to the previous claim **characterized by** the step a) comprising the following steps:
i. placing the sample in Golgi-Cox solution and maintain it in the dark for at least 15 days;
ii. placing the sample in a 30% sucrose solution, previously refreshed;
iii. maintaining the sample under refrigeration at a temperature of 4 ° C in the dark for 2-5 days;
iv. dipping the sample in distilled water for 15 minutes, then in ammonium hydroxide for 5 minutes in the dark;
v. washing the samples with distilled water and dipping the samples in the Kodak fixing solution;
vi. washing the samples with distilled water and dipping the samples in phosphate buffered saline solution.

4. Methodology according to the previous claim **characterized by** in step ii) of step a) the solution is cooled to a temperature of 4°C.

5. Methodology according to the claim 3 **characterized by** in step v) of step a) the samples are submerged in the fixing solution for at least 20 minutes.

6. Methodology according to the claim 2 **characterized in that** the step b) comprises the following steps:
i. placing the samples in citrate buffer solution;
ii. heating the samples for 5 minutes;
iii. cooling the specimens to room temperature;
iv. dipping the samples in Tris buffer solution three times;
v. incubate the samples with primary antibody for BrdU;
vi. removing the primary antibody solution and dipping the samples three times in TBS, 10 minutes each;
vii. incubating the sample with secondary antibody for 2 hours at room temperature;
viii. removing the secondary antibody and dipping the samples in TBS three times;
ix. incubating the samples in DAPI at the concentration of 1µg/ml in a solution of TBS for 10 minutes at room temperature and then the samples were dipped 3 times in TBS, 3 minutes each.

7. Methodology according to the previous claim **characterized by** in step i) the buffer solution has a concentration of 10 mM and pH 6.

8. Methodology according to the claim 6 **characterized by** the heating in step ii) occurs at a power between 750 and 900 watts.

9. Methodology according to the claim 6 **characterized by** in step iv) the samples remain immersed in Tris buffer for 10 minutes each time.

10. Methodology according to the claim 6 **characterized by** in step v) the samples are incubated overnight at 4°C.

11. Methodology according to the claim 6 **characterized by** the primary antibody for BrdU is used at a dilution of 1:50.

12. Methodology according to the claim 6 **characterized by** in step viii) the samples remain immersed in TBS 10 minutes each wash.

13. Methodology according to the claims 4-5 **characterized by** the secondary antibody is used at the dilution of 1:1000.

14. Using the methodology described in the previous claims **characterized by** being used to analyze the three-dimensional structure of the dendritic arborization of neurons and spines shape.

15. Using the methodology according to the previous claim **characterized by** being applied in physiological or pathological conditions in which neurogenesis and synaptic plasticity may be affected.

## Patentansprüche

1. Methodologie für die Markierung von Neuronen, **dadurch gekennzeichnet, dass** die Golgi-Cox-Imprägnierungstechnik und die Technik der Immunhistochemie für Bromdesoxyuridin (BrdU) gleichzeitg kombiniert werden.

2. Methodologie nach dem vorhergehendem Anspruch, **dadurch gekennzeichnet, dass** diese folgende Schritte umfasst:
a) Etablieren der Golgi-Cox-Imprägnierungstechnik in Neuronenproben;
b) Etablieren einer Immunohistochemie für BrdU in den gleichen zuvor mit Golgi-Cox imprägnierten Proben;
c) Analyse der Proben.

3. Methodologie nach dem vorhergehendem Anspruch, **dadurch gekennzeichnet, dass** Schritt a) folgende Schritte umfasst:
i) Platzieren der Probe in der Golgi-Cox-Lösung und Erhalten derselben im Dunkeln für mindestens 15 Tage;
ii) Platzieren der Probe in einer 30%igen Saccharoselösung, welche zuvor aufgefrischt wurde;
iii) Erhalten der Probe unter Kühlung bei einer Temperatur von 4 °C im Dunkeln für 2 - 5 Tage;
iv) Eintauchen der Probe in destilliertes Wasser für 15 Minuten, dann in Ammoniumhydroxid für 5 Minuten im Dunkeln;
v) Waschen der Proben mit destilliertem Wasser und Eintauchen der Proben in der Kodak-Fixierlösung;
vi) Waschen der Proben mit destilliertem Wasser und Eintauchen der Proben in einer Phosphatgepufferten Salzlösung.

4. Methodologie nach dem vorhergehendem Anspruch, **dadurch gekennzeichnet, dass** in Schritt ii) von Schritt a) die Lösung auf eine Temperatur von 4 °C abgekühlt wird.

5. Methodologie nach Anspruch 3, **dadurch gekennzeichnet, dass** in Schritt v) von Schritt a) die Proben für mindestens 20 Minuten in der Fixierlösung eingetaucht werden.

6. Methodologie nach Anspruch 2, **dadurch gekennzeichnet, dass** Schritt b) folgende Schritte umfasst:
i) Platzieren der Proben in einer Citratpufferlösung;
ii) Erhitzen der Proben für 5 Minuten;
iii) Abkühlung der Proben auf Raumtemperatur;
iv) Dreimaliges Eintauchen der Proben in Tris-Pufferlösung;
v) Inkubieren der Proben mit einem primären Antikörper für BrdU;
vi) Entfernen der primären Antikörperlösung und dreimaliges Eintauchen der Proben in TBS, jeweils 10 Minuten lang;
vii) Inkubieren der Probe mit einem sekundären Antikörper für 2 Stunden bei Raumntemperatur;
viii) Entfernen der sekundären Antikörperlösung und dreimaliges Eintauchen der Proben in TBS;
ix) Inkubieren der Proben in DAPI in einer Konzentration von 1 µg/ml in einer TBS-Lösung für 10 Minuten bei Raumtemperatur und darauffolgend wurden die Proben 3 mal in TBS eingetaucht, jeweils 3 Minuten lang.

7. Methodologie nach dem vorhergehendem Anspruch, **dadurch gekennzeichnet, dass** in Schritt i) die Pufferlösung eine Konzentration von 10 nM und pH 6 aufweist.

8. Methodologie nach Anspruch 6, **dadurch gekennzeichnet, dass** das Erhitzen in Schritt ii) bei einer Leistung zwischen 750 Watt und 900 Watt erfolgt.

9. Methodologie nach Anspruch 6, **dadurch gekennzeichnet, dass** in Schritt iv) die Proben jeweils 10 Minuten lang in Tris-Puffer eingetaucht bleiben.

10. Methodologie nach Anspruch 6, **dadurch gekennzeichnet, dass** in Schritt v) die Proben über Nacht bei 4 °C inkubiert werden.

11. Methodologie nach Anspruch 6, **dadurch gekennzeichnet, dass** der primäre Antikörper für BrdU bei einer Verdünnung von 1:50 verwendet wird.

12. Methodologie nach Anspruch 6, **dadurch gekennzeichnet, dass** in Schritt viii) die Proben jeweils 10 Minuten pro Waschgang in TBS eingetaucht bleiben.

13. Methodologie nach Ansprüchen 4 - 5, **dadurch gekennzeichnet, dass** der sekundäre Antikörper bei einer Verdünnung von 1:1000 verwendet wird.

14. Verwendung der in den vorhergehenden Ansprüchen beschriebenen Methodologie, **dadurch gekennzeichnet, dass** diese dazu verwendet wird, die dreidimensionale Struktur der dendritischen Verzweigung von Neuronen und Stachelform, zu analysieren.

15. Verwendung der in den vorhergehenden Ansprüchen beschriebenen Methodologie, **dadurch gekennzeichnet, dass** diese in physiologischen oder pathologischen Zuständen, in denen die Neurogenese und die synaptische Plastizität beeinträchtigt werden können, verwendet wird.

## Revendications

1. Méthodologie de marquage de neurones, **caractérisée en ce qu'**elle combine simultanément la technique d'imprégnation Golgi-Cox et la technique de l'immunohistochimie pour la bromodéoxyuridine (BrdU).

2. Méthodologie selon la revendication précédente, **caractérisée en ce qu'**elle comprend les étapes suivantes:
a) Établir la technique d'imprégnation Golgi-Cox dans des échantillons de neurones;
b) Établir une immunohistochimie pour BrdU dans les mêmes échantillons préalablement imprégnés de Golgi-Cox;
c) L'analyse des échantillons.

3. Méthodologie selon la revendication précédente, **caractérisée en ce que** l'étape a) comprend les étapes suivantes:
i) Placer l'échantillon dans une solution Golgi-Cox et la maintenir dans l'obscurité pendant au moins 15 jours;
ii) Placer l'échantillon dans une solution de saccharose à 30%, préalablement régénérée;
iii) Maintenir de l'échantillon sous réfrigération à une température de 4 °C dans l'obscurité pendant 2 - 5 jours;
iv) Plonger l'échantillon dans de l'eau distillée pendant 15 minutes, puis dans de l'hydroxyde d'ammonium pendant 5 minutes dans l'obscurité;
v) Laver les échantillons avec de l'eau distillée et plonger les échantillons dans la solution de fixage Kodak;
vi) Laver les échantillons avec de l'eau distillée et plonger les échantillons dans une solution saline tamponnée au phosphate.

4. Méthodologie selon la revendication précédente, **caractérisée en ce que** dans l'étape ii) de l'étape a) la solution est refroidie à une température de 4 °C.

5. Méthodologie selon la revendication 3, **caractérisée en ce que** dans l'étape v) de l'étape a) les échantillons sont submergés dans la solution de fixation pendant au moins 20 minutes.

6. Méthodologie selon la revendication 2, **caractérisée en ce que** l'étape b) comprend les étapes suivantes:
i) Placer les échantillons dans une solution de tampon au citrate;
ii) Chauffer les échantillons pendant 5 minutes;
iii) Refroidir les échantillons jusqu'à température ambiante;
iv) Plonger les échantillons dans une solution de tampon Tris trois fois;
v) Incuber les échantillons avec un anticorps primaire pour BrdU;
vi) Enlever la solution d'anticorps primaire et plonger les échantillons trois fois dans du TBS, à 10 minutes chacune;
vii) Incuber l'échantillon avec un anticorps secondaire pendant 2 heures à la température ambiante;
viii) Enlever l'anticorps secondaire et plonger les échantillons dans du TBS trois fois;
ix) Incuber les échantillons dans du DAPI à la concentration de 1 µg/ml dans une solution de TBS pendant 10 minutes à température ambiante puis les échantillons ont été plongés 3 fois dans du TBS, à 3 minutes chacune.

7. Méthodologie selon la revendication précédente, **caractérisée en ce que** dans l'étape i) la solution tampon a une concentration de 10 mM et pH 6.

8. Méthodologie selon la revendication 6, **caractérisée en ce que** le chauffage dans l'étape ii) a lieu à une puissance comprise entre 750 watts et 900 watts.

9. Méthodologie selon la revendication 6, **caractérisée en ce que** dans l'étape iv) les échantillons restent immergés dans du tampon Tris pendant 10 minutes à chaque fois.

10. Méthodologie selon la revendication 6, **caractérisée en ce que** dans l'étape v) les échantillons sont incubés pendant la nuit à 4 °C.

11. Méthodologie selon la revendication 6, **caractérisée en ce que** l'anticorps primaire pour BrdU est utilisé à une dilution de 1:50.

12. Méthodologie selon la revendication 6, **caractérisée en ce que** dans l'étape viii) les échantillons restent immergés dans du TBS 10 minutes à chaque lavage.

13. Méthodologie selon les revendications 4-5, **caractérisée en ce que** l'anticorps secondaire est utilisé à la dilution de 1: 1000.

14. Utilisation de la méthodologie décrite dans les revendications précédentes utilisée pour analyser la structure tridimensionnelle de l'arborisation dendritique des neurones et la forme des épines.

15. Utilisation de la méthodologie décrite dans le revendications précédentes appliquée dans des conditions physiologiques ou pathologiques dans lesquelles la neurogenèse et la plasticité synaptique peuvent être affectées.
